# EUROPÄISCHE PATENTSCHRIFT

(11) **EP 3 827 729 B1**
(45) Veröffentlichungstag und Bekanntmachung des Hinweises auf die Patenterteilung: **19.07.2023**
(21) Anmeldenummer: 20209134.4
(22) Anmeldetag: 23.11.2020
(51) Int. Cl.: A61B 1/00, A61B 1/005, G02B 23/24

(54) **MULTIFUNKTIONSKAMERA**
MULTIFUNCTION CAMERA
CAMÉRA MULTIFONCTION

(30) Priorität: 29.11.2019 AT 5020919 U
(43) Veröffentlichungstag der Anmeldung: 02.06.2021
(73) Patentinhaber: Walter Werkzeuge Salzburg GmbH, 5081 Anif (AT)
(72) Erfinder: Rothenberger, Helmut, 60323 Frankfurt am Main (DE)
(74) Vertreter: Schwarz & Partner Patentanwälte GmbH

(56) Entgegenhaltungen:
- CN-U- 202 548 434
- CN-U- 209 590 401
- DE-B4-112009 000 536
- US-A1- 2007 226 258
- US-A1- 2008 175 301

## Beschreibung

Die Erfindung betrifft ein Multifunktionskamera-Taschen-Endoskop umfassend einen Grundkörper, eine Inspektionskamera, welche am Ende eines am Grundkörper anschließbaren Kabels angebracht ist, einen Distanzmesser, einen Umgebungstemperaturmesser, einen Laser Pointer, und eine Taschenlampe.

### HINTERGRUND DER ERFINDUNG

Endoskope finden ihre Anwendung nicht nur im medizinischen Bereich, sondern auch im technischen Bereich, wo oft schwer zugängliche Stellen von Maschinen, Leitungen, Rohren oder dergleichen untersucht werden müssen.

Gängige Endoskope verfügen über einen Bildschirm, welcher die von der Kamera aufgenommenen Bilder überträgt. Diese Endoskope sind sehr oft klobig und passen somit nicht in Arbeitskleidung-Taschen. Außerdem ist die einzige Funktion solcher Endoskope meist das Übertragen von Live-Videos bzw. das Aufnehmen und spätere Abspielen von Fotos.

Endoskope sind präzise Messwerkzeuge, die nicht nur in schwer zugänglichen Bereichen eingesetzt werden, sondern auch unter erschwerten Schmutz- und Gefahrenbedingungen präzise Ergebnisse liefern müssen.

Die Druckschrift CN209590401U offenbart ein Multifunktionskamera-Taschen-Endoskop gemäß dem Obergriff des Anspruchs 1.

### KURZBESCHREIBUNG DER ERFINDUNG

Die Erfindung wird durch die unabhängigen Ansprüche 1 und 13 definiert. Aufgabe der vorliegenden Erfindung ist daher ein Endoskop bereitzustellen, welches gleichermaßen über kompakte Außenmaße verfügt und dennoch unter den erschwerten Einsatzbedingungen gegenüber dem Stand der Technik bessere Messergebnisse liefert.

Gelöst wird diese Aufgabe durch ein Multifunktionskamera-Taschen-Endoskop umfassend einen Grundkörper, eine Inspektionskamera, welche am Ende eines am Grundkörper anschließbaren Kabels angebracht ist, einen Distanzmesser, einen Umgebungstemperaturmesser, einen Laser Pointer, und eine Taschenlampe. An der Vorderseite des Grundkörpers des erfindungsgemäßen Endoskops ist eine Flüssigkristallanzeige (LCD) angebracht.

Die Leistung des Laser Pointers, welcher sich an der Oberseite des Grundkörpers befindet, entspricht dabei vorzugsweise der Laser-Klasse 2. Für die Taschenlampe befindet sich ebenfalls an der Oberseite des Grundkörpers eine Weißlicht-LED.

Der Grundkörper des Multifunktionskamera/Taschen-Endoskops entspricht dabei in der Form etwa gängigen Smartphones und kann daher leicht transportiert werden. Diese kompakte Bauweise macht das Endoskop zu einem praktischen Handgerät, das auch leicht in einer Hosentasche Platz hat.

Mit einem erfindungsgemäßen Endoskop können daher verbesserte Messergebnisse erzielt werden und es muss nicht für unterschiedliche Untersuchungen ein extra Messgerät verwendet werden, was diese Untersuchungen schneller und effizienter für den Inspekteur macht. Des Weiteren spielt auch der wirtschaftliche Faktor eine Rolle, da die Anschaffung mehrere Messgeräte kostspielig werden kann. Es hat sich herausgestellt, dass ein solches Endoskop als Synergieeffekt genauere Messergebnisse liefert als mehrere Einzelgeräte.

Des Weiteren ist der Grundkörper vorzugsweise an der Unterseite mit einem USB-Anschluss ausgestattet. Anhand dieses Anschlusses kann das erfindungsgemäße Endoskop mithilfe eines passenden Verbindungskabels mit jedem Computer verbunden werden. Somit können zwischen dem Endoskop und dem Computer Daten übertragen werden. Bevorzugt handelt es sich bei dem USB-Anschluss um einen USB 2.0 oder USB 3.0.

In einer Ausführungsvariante ist im Multifunktionskamera/Taschen-Endoskop ein integrierter Speicher verbaut, welcher erlaubt, Fotos zu speichern. Dabei können vorzugsweise bis zu 100 Bilder gespeichert werden.

Der integrierte Distanzmesser des Endoskops erlaubt eine messbare Distanz von vorzugsweise 5 cm bis 20 m. Bei dem Distanzmesser handelt es sich um einen Laserentfernungsmesser unter Zuhilfenahme des Laser Pointers. Hierfür kann einfach der integrierte Laser Pointer als Laserstrahl verwendet werden. Die gemessene Distanz kann am LCD-Display angezeigt werden. Die Distanz kann dabei in verschiedenen Einheiten angegeben werden, bevorzugt in Meter (m), Inch (in) und Fuß (ft). Nach der Distanzmessung wird der Laser Pointer automatisch ausgeschalten.

Bei dem integrierten Umgebungstemperaturmesser handelt es sich vorzugsweise um ein Infrarot Thermometer. Das Infrarot Thermometer kann eine Temperatur von -20 °C bis 350 °C messen und verwendet hierfür den IR-Sensor (Infrarot-Sensor), welcher mithilfe des Laser Pointers den Messpunkt anvisiert. Die Messoptik des Infrarot Thermometers ist über das Verhältnis von Messabstand zur Größe des Messflecks mit vorzugsweise D:S = 8:1 gegeben. Nach der Messung ist die Temperatur am LCD-Display anzeigbar, wobei die Temperatur in Grad Celsius (°C) und Fahrenheit (°F) angegeben wird.

Bevorzugt handelt es sich bei der LCD-Anzeige um einen Dünnfilmtransistor-Display, wobei das Display vorzugsweise eine Größe von 4 bis 5, beispielsweise 4,3 Zoll und eine Auflösung von 300 bis 600 × 200 bis 400, beispielsweise 480 × 272 Bildpunkten aufweist.

Des Weiteren kann die LCD-Anzeige mehrere Anpassungsfunktionen umfassen, dazu gehören eine Zoom-Funktion, eine Rotations-Funktion und eine Hintergrundlicht-Funktion. Die Zoom-Funktion weist vorzugsweise vier Stufen auf, genauer gesagt 1X, 2X, 3X und 4X. Nach der Zoom Einstellung wird das entsprechende Abbildungsverhältnis vorzugsweise im rechten oberen Eck an der LCD-Anzeige angezeigt. Die Rotations-Funktion weist vorzugsweise die folgenden vier Rotationen bzw. Spiegelungen auf: normal, 180° Drehung, Spiegelung der 180° Drehung und Spiegelung von normal. Die LCD-Anzeige ist nach Verwendung der Rotations-Funktion entsprechend gedreht oder gespiegelt. Die Hintergrundlicht-Funktion weist vorzugsweise sieben Stufen auf. Mit jeder Stufe ändert sich die Helligkeit des LCD-Displays.

In einer weiteren Ausführungsvariante weist das erfindungsgemäße Multifunktionskamera/Taschen-Endoskop eine Einschub-Rille zwischen der Vorder- und Rückseite des Grundkörpers auf. Diese Einschub-Rille kann geöffnet werden, indem Vorder- und Rückseite des Grundkörpers durch Lösen der Verriegelung auseinandergezogen werden.

Nach dem Lösen der Verriegelung öffnet sich die Einschub-Rille, in welcher das Kabel samt Inspektionskamera aufbewahrbar ist. Während der Verwendung der Inspektionskamera kann die Verriegelung wieder geschlossen werden. Nach der Verwendung kann nach abermaliger Öffnung der Verriegelung das Kabel mitsamt Inspektionskamera wieder in der Einschub-Rille aufgewickelt werden.

Bevorzugt umfasst die Inspektionskamera LEDs, wobei die Helligkeit der LEDs vorzugsweise in sieben Stufen regelbar ist, wobei bei der ersten Stufe alle LEDs aus sind. Die Helligkeitsstufen werden geregelt durch Hinzu- bzw. Wegschalten von LEDs, daher umfasst die Inspektionskamera vorzugsweise 6 LEDs.

In einer weiteren Ausführungsvariante des Multifunktionskamera/Taschen-Endoskops umfasst der Grundkörper des Endoskops neun Schalter zur Steuerung der verschiedenen Funktionen und Anpassungen. Dazu gehören folgende Schalter:
- Funktionsschalter "Wiedergabe / Displayhelligkeit"
- Funktionsschalter "Löschen / Ausrichtung"
- Funktionsschalter "LED Arbeitsleuchte / Laserpointer"
- Funktionsschalter "Zoom / rauf"
- Funktionsschalter "LED Helligkeit / runter"
- Funktionsschalter "Ein- / Ausschalter"
- Auswahlschalter "Temperaturmessung"
- Auswahlschalter "Kameraaufnahme"
- Auswahlschalter "Entfernungsmessung"

Dabei erlaubt der Funktionsschalter "Wiedergabe / Displayhelligkeit" den Wechsel in den Wiedergabe-Modus und bei längerem Drücken die Anpassung der Helligkeit des LCD-Displays. Der Funktionsschalter "Löschen / Ausrichtung" ermöglicht ein vierstufiges Rotieren des LCD-Displays, einen Farbwechsel bzw. Schwarz-Weiß-Wechsel sowie das Löschen einzelner Fotos. Mit dem Funktionsschalter "LED Arbeitsleuchte / Laserpointer" kann einerseits die Taschenlampe und andererseits auch der Laser Pointer ein- und ausgeschalten werden. Der Funktionsschalter "Zoom / rauf" verändert das Abbildungsverhältnis des LCD-Displays, stellt ein Standbild ein und schaltet bei der Wiedergabe von aufgenommenen Fotos auf das vorherige Foto zurück. Der Funktionsschalter "LED Helligkeit / runter" erlaubt die Helligkeitseinstellung der Kamera-LEDs, schaltet in den Vergleichs-Modus mit geteiltem Bildschirm und bei der Wiedergabe von Fotos auf das nächste Foto. Der Funktionsschalter "Ein- / Ausschalter" schaltet das Endoskop ein bzw. aus. Der Auswahlschalter "Temperaturmessung" ermöglicht die Temperatur-Messung und der Auswahlschalter "Kameraaufnahme" das Übertragen des Live-Videos auf den LCD-Display, das Aufnehmen von Fotos und das Löschen aller Fotos. Der Auswahlschalter "Entfernungsmessung" ermöglicht das Messen einer Distanz.

In einer weiteren Ausführungsvariante befindet sich vorzugsweise an der Rückseite des Grundkörpers ein Batteriefach mit zumindest einer Batterie zur Stromversorgung des Multifunktionskamera/Taschen-Endoskops. Besonders bevorzugt sind vier AA Batterien im Batteriefach einlegbar.

Außerdem erlaubt das erfindungsgemäße Multifunktionskamera/Taschen-Endoskops eine Verwendung in unterschiedlichen Modi. Dazu wird das Endoskop zuerst über den "Ein- / Ausschalter" eingeschalten und kann danach in einer der Betriebsarten
- Modus "Entfernungsmessung"
- Modus "Kameraaufnahme"
- Modus "Temperaturmessung"
- Modus "USB Übertragung" (automatisch bei Anschluss des USB-Kabels)
verwendet werden.

Im Kamera-Modus gibt es bevorzugt folgende zusätzliche Betriebsarten:
- Liveansicht-Modus,
- Vergleichs-Modus,
- Wiedergabe-Modus,
- Lösch-Modus.

Dabei kann im Liveansicht-Modus das von der Inspektionskamera aufgenommene Video direkt am LCD-Display angezeigt und Fotos aufgenommen werden. Im Vergleichs-Modus kann der LCD-Display in einen ersten Bereich und einen zweiten Bereich geteilt werden, wobei im ersten Bereich ein Standbild und im zweiten Bereich entweder eine Liveübertragung der Inspektionskamera oder ebenfalls ein Standbild darstellbar ist. Außerdem können im Wiedergabe-Modus die aufgenommenen Fotos am LCD-Display betrachtet werden und im Lösch-Modus alle aufgenommenen Fotos gelöscht werden. Bevorzugt können auch im Wiedergabe-Modus einzelne Fotos gelöscht werden.

In einer Ausführungsvariante kann am Grundkörper (z.B. an der Unterseite des Grundkörpers) eine Anschlussstelle für ein Stativ vorgesehen sein. Dies ermöglicht eine stabile Aufstellung des Geräts.

### DETAILLIERTE BESCHREIBUNG DER ERFINDUNG

Weitere Details und Vorteile der Erfindung ergeben sich aus den nachfolgenden Figuren und der dazugehörigen Figurenbeschreibung.

Dabei zeigt:
Fig. 1 eine Vorderansicht des erfindungsgemäßen Multifunktionskamera/Taschen-Endoskops.
Fig. 2 eine Seitenansicht des erfindungsgemäßen Multifunktionskamera/Taschen-Endoskops mit geöffneter Einschub-Rille.
Fig. 3 eine Ansicht auf die Oberseite des erfindungsgemäßen Multifunktionskamera/Taschen-Endoskops.
Fig. 4 eine Darstellung der Hinterseite des erfindungsgemäßen Multifunktionskamera/Taschen-Endoskops mit Batteriefach.

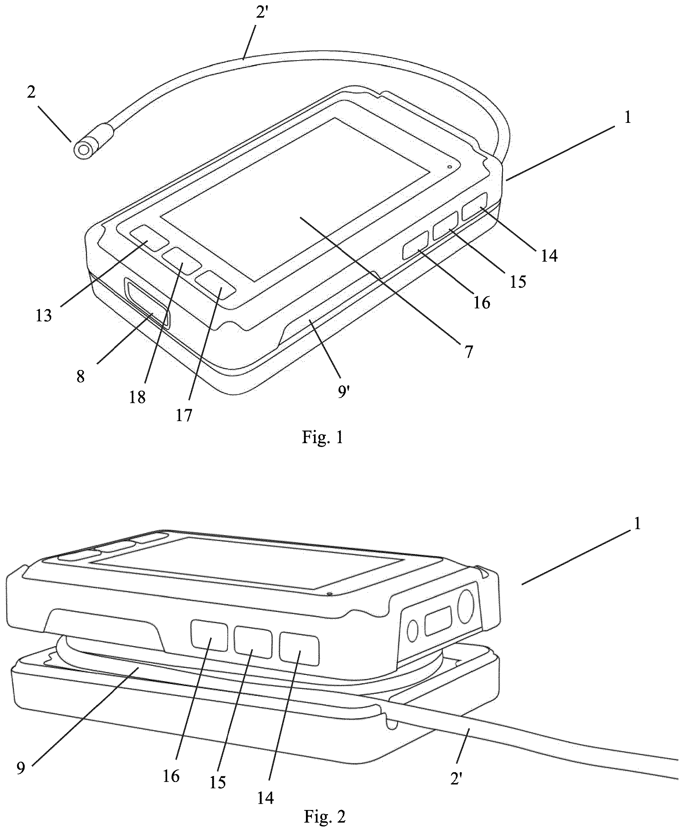

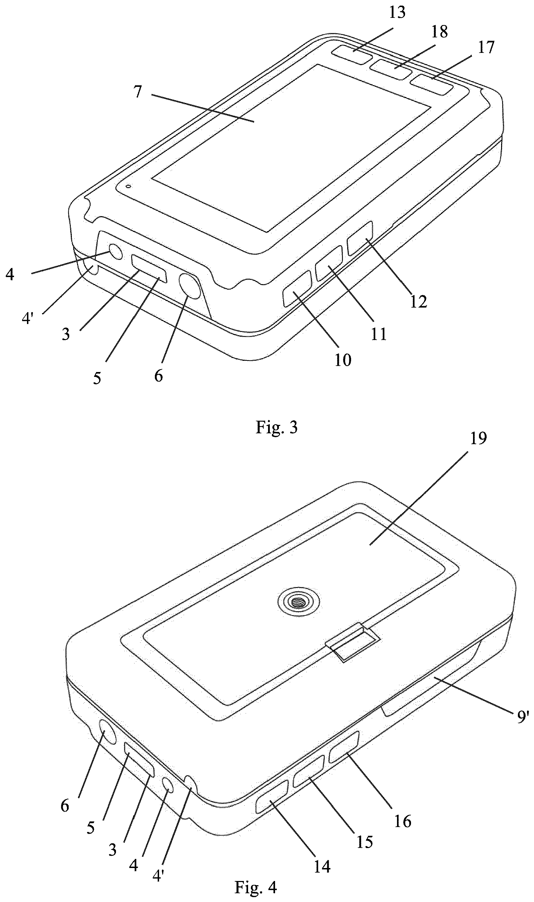

Das erfindungsgemäße Multifunktionskamera/Taschen-Endoskop umfasst wie in Fig. 1 und Fig. 3 ersichtlich einen Grundkörper 1, eine Inspektionskamera 2, welche am Ende eines am Grundkörper 1 anschließbaren Kabels 2` angebracht ist, einen Distanzmesser 3, einen Umgebungstemperaturmesser 4, einen Laser Pointer 5 und eine Taschenlampe 6. An der

Vorderseite des Grundkörpers 1 ist, wie in der Abbildung des erfindungsgemäßen Endoskops in Fig. 1 dargestellt, die Flüssigkristallanzeige (LCD) 7 angebracht.

Außerdem befindet sich an der Unterseite des Grundkörpers 1 der in Fig. 1 gezeigte zumindest eine USB-Anschluss 8.

Fig. 3 zeigt die Oberseite des Multifunktionskamera/Taschen-Endoskops, an welcher sich unter anderem der Distanzmesser 3 und der Laser Pointer 5 befinden. In dieser Ausführungsvariante sind der Distanzmesser 3 bzw. Laser Pointer 5 mittig an der Oberseite des Grundkörpers 1 angeordnet, wobei sich direkt daneben der Umgebungstemperaturmesser 4 mit dem IR-Sensor befindet. Außerdem ist seitlich links bzw. rechts vom Distanzmesser 3 bzw. Laser Pointer 5 und dem Umgebungstemperaturmesser 4 eine Anschlussmöglichkeit für einen externen Temperaturfühler 4` und die Taschenlampe 6 angeordnet.

Der Distanzmesser 3 ist ein Laserentfernungsmesser mit einer Reichweite von 5 cm bis 20 m, welcher den Laserstrahl zur Messung der Distanz verwendet. Nach jeder Messung wird die Distanz am LCD-Display 7 angezeigt.

Der Umgebungstemperaturmesser 4 mit IR-Sensor ist ein Infrarot Thermometer mit einem Temperaturbereich von -20 °C bis 350 °C. Auch das Infrarot Thermometer verwendet zur Temperaturmessung den IR-Sensor und es wird auch nach jeder Messung die Temperatur am LCD-Display 7 angezeigt.

Bei der Taschenlampe 5 handelt es sich um eine Weißlicht-LED.

Die in Fig.1 gezeigte Flüssigkristallanzeige 7 ist ein Dünnfilmtransistor-Display mit einer Größe von 4,3 Zoll und einer Auflösung von 480 × 272 Bildpunkten.

Außerdem ist in der Seitenansicht der erfindungsgemäßen Multifunktionskamera/Taschen-Endoskops in Fig. 2 die Einschub-Rille 9 zwischen der Vorder- und Rückseite des Grundkörpers 1 erkennbar. In Fig. 1 ist die Rille 9 geschlossen und nur die Verriegelung 9` ist sichtbar. Durch Lösen der Verriegelung 9` öffnet sich die Einschub-Rille 9, wie in Fig. 2 gezeigt.

In dieser Einschub-Rille 9 kann das Kabel 2' samt Inspektionskamera 2 aufgewickelt werden. Die Rille 9 ist dabei so tief ausgeführt, dass nachdem das Kabel 2` aufgewickelt wurde die Vorder- und Rückseite des Grundkörpers 1 wieder zusammengedrückt werden können und die Verriegelung 9` geschlossen werden kann. Auch während der Verwendung der Inspektionskamera 2 kann die Verriegelung 9` für eine komfortablere Handhabung des Endoskops geschlossen werden.

Bevorzugt umfasst die Inspektionskamera 2 mehrere LEDs. Über diese LEDs lässt sich auch die Helligkeit direkt an der Kamera einstellen. In einem Ausführungsbeispiel umfasst die Inspektionskamera 2 sechs LEDs, das heißt die Helligkeit lässt sich in sieben Stufen regeln. Genauer gesagt ist in der ersten Stufe keine der LEDs, in der zweiten Stufe eine LED, usw. und in der siebten Stufe alle LEDs an. Damit kann auch in dunkeln Hohlräumen mit der Inspektionskamera 2 des erfindungsgemäßen Endoskops gefilmt werden.

Zur Stromversorgung des Endoskops befindet sich wie in Fig. 4 gezeigt ein Batteriefach 19 an der Rückseite des Grundkörpers 1. Im in Fig. 4 dargestellten Endoskop können dabei im Batteriefach 19 vier AA Batterien eingelegt werden. Die vier Batterien erlauben eine möglichst lange Laufzeit des Endoskops, auch in Bezug auf das Gewicht des Endoskops, welches durch die vier Batterien nicht drastisch erhöht wird.

Zur Steuerung der Funktionen und Verwendung der unterschiedlichen Modi des Multifunktionskamera/Taschen-Endoskops befinden sich am Grundkörper 1 des Endoskops mehrere Schalter, wie in den Figuren 1 bis 4 dargestellt. Auf der Vorderseite des Endoskops befinden sich vorzugsweise im unteren Bereich, das heißt unter dem LCD-Display 7, die Auswahlschalter Entfernungsmessung 13, Temperaturmessung 17 und Kameraaufnahme 18. Mit diesen Auswahlschaltern wird daher der Distanzmesser 3, der Umgebungstemperaturmesser 4 oder die Inspektionskamera 2 aktiviert.

Außerdem befinden sich auf einer Seite des Endoskops die Funktionsschalter Zoom / rauf 14, LED Helligkeit / runter 15 und Ein- / Ausschalter 16. Auf der anderen Seite befinden sich die drei Funktionsschalter Wiedergabe / Displayhelligkeit 10, Löschen / Ausrichtung 11 und LED Arbeitsleuchte / Laserpointer 12.

Mit dem Funktionsschalter Ein- / Ausschalter 16 kann das Endoskop bei Verwendung eingeschalten werden. Danach kann das Endoskop in einer der Betriebsarten Kameraaufnahme, Temperaturmessung, Entfernungsmessung oder USB Übertragung verwendet werden.

Im Modus Kameraaufnahme kann eine der vier Betriebsarten Liveansicht-Modus, Vergleichs-Modus, Wiedergabe-Modus und Lösch-Modus mit dem dazugehörigen Schalter gewählt werden.

Im Liveansicht-Modus wird das von der Inspektionskamera 2 aufgenommene Video direkt am LCD-Display 7 angezeigt. Außerdem können Fotos aufgenommen werden. Im Vergleichs-Modus ist der LCD-Display 7 in einen ersten Bereich und einen zweiten Bereich geteilt, wobei im ersten Bereich ein Standbild und im zweiten Bereich entweder eine Liveübertragung der Inspektionskamera 2 oder ebenfalls ein Standbild dargestellt werden kann. Im Wiedergabe-Modus können die aufgenommenen Fotos am LCD-Display 7 betrachtet werden. Im Lösch-Modus können alle aufgenommenen Fotos gelöscht werden, wobei vorzugsweise auch im Wiedergabe-Modus einzelne Fotos löschbar sind.

Außerdem ist in Fig. 4 an der Unterseite des Grundkörpers 1 eine Anschlussstelle für ein Stativ erkennbar. Dies ermöglicht eine stabile Aufstellung des Geräts.

## Patentansprüche

1. Multifunktionskamera-Taschen-Endoskop umfassend
• einen Grundkörper (1),
• eine Inspektionskamera (2), welche am Ende eines am Grundkörper (1) angeschlossenen Kabels (2') angebracht ist,
• einen Distanzmesser (3),
• einen Umgebungstemperaturmesser (4),
• und eine Taschenlampe (6),
**dadurch gekennzeichnet, dass** ein Laser Pointer (5) vorgesehen ist und dass an der Vorderseite des Grundkörpers (1) eine Flüssigkristallanzeige (LCD) (7) angebracht ist, wobei die Flüssigkristallanzeige (7)
• eine Zoom-Funktion,
• eine Rotations-Funktion und
• eine Hintergrundlicht-Funktion
umfasst, wobei die Zoom-Funktion vorzugsweise vier Stufen aufweist, wobei die Rotations-Funktion vorzugsweise vier Rotationen bzw. Spiegelungen aufweist, wobei die Hintergrundlicht-Funktion vorzugsweise sieben Stufen aufweist.

2. Multifunktionskamera-Taschen-Endoskop nach Anspruch 1, wobei der Grundkörper (1) über einen Anschluss für einen externen Temperaturfühler (4`) verfügt.

3. Multifunktionskamera-Taschen-Endoskop nach einem der Ansprüche 1 oder 2, wobei der Grundkörper (1) über zumindest einen USB-Anschluss (8) verfügt.

4. Multifunktionskamera-Taschen-Endoskop nach einem der Ansprüche 1 bis 3, **gekennzeichnet durch** einen integrierten Speicher, wobei der integrierte Speicher vorzugsweise bis zu 100 Bilder speichern kann.

5. Multifunktionskamera-Taschen-Endoskop nach einem der Ansprüche 1 bis 4, wobei mit dem Distanzmesser (3) eine Distanz von vorzugsweise 5 cm bis 20 m messbar ist.

6. Multifunktionskamera-Taschen-Endoskop nach einem der Ansprüche 1 bis 5, wobei der Umgebungstemperaturmesser (4) ein Infrarot Thermometer ist, wobei das Infrarot Thermometer eine Temperatur von -20 °C bis 350 °C misst, wobei die Messoptik des Thermometers (4) über das Verhältnis von Messabstand zur Größe des Messflecks mit vorzugsweise D:S = 8:1 gegeben ist.

7. Multifunktionskamera-Taschen-Endoskop nach einem der Ansprüche 1 bis 6, wobei es sich bei der Flüssigkristallanzeige (7) um einen Dünnfilmtransistor-Display handelt, wobei der Display (7) vorzugsweise eine Größe von 4,3 Zoll und eine Auflösung von 480 × 272 Bildpunkten aufweist.

8. Multifunktionskamera-Taschen-Endoskop nach einem der Ansprüche 1 bis 7, **gekennzeichnet durch** eine Einschub-Rille (9) zwischen der Vorder- und Rückseite des Grundkörpers (1), wobei sich die Einschub-Rille (9) öffnet, indem Vorder- und Rückseite des Grundkörpers (1) durch Lösen der Verriegelung (9`) auseinandergezogen werden.

9. Multifunktionskamera-Taschen-Endoskop nach einem der Ansprüche 1 bis 8, wobei die Inspektionskamera (2) LEDs umfasst, wobei die Helligkeit der LEDs vorzugsweise in sieben Stufen regelbar ist, wobei vorzugsweise bei der ersten Stufe alle LEDs aus sind.

10. Multifunktionskamera-Taschen-Endoskop nach einem der Ansprüche 1 bis 9, wobei die Inspektionskamera (2) und das Kabel (2') in der Einschub-Rille (9) verstaubar sind.

11. Multifunktionskamera-Taschen-Endoskop nach einem der Ansprüche 1 bis 10, wobei der Grundkörper (1) des Endoskops die Funktionsschalter
• Wiedergabe / Displayhelligkeit (10)
• Löschen / Ausrichtung (11)
• LED Arbeitsleuchte / Laserpointer (12)
• Zoom / rauf (14)
• LED Helligkeit / runter (15)
• Ein- / Ausschalter (16)
und die Auswahlschalter
• Entfernungsmessung (13)
• Temperaturmessung (17)
• Kameraaufnahme (18)
umfasst.

12. Multifunktionskamera-Taschen-Endoskop nach einem der Ansprüche 1 bis 11, **gekennzeichnet durch** ein Batteriefach (19) mit zumindest einer Batterie zur Stromversorgung, wobei vorzugsweise vier AA Batterien im Batteriefach (19) einlegbar sind.

13. Verwendung eines Multifunktionskamera-Taschen-Endoskops nach einem der Ansprüche 1 bis 12, wobei das Endoskop zuerst über einen Funktionsschalter Ein- / Ausschalter (16) eingeschalten wird, wobei das Endoskop danach in einer der Betriebsarten
• Modus Kameraaufnahme,
• Modus Temperaturmessung,
• Modus Entfernungsmessung,
• Modus USB Übertragung
einsetzbar ist.

14. Verwendung eines Multifunktionskamera-Taschen-Endoskop nach Anspruch 13 im Modus Kameraaufnahme, welcher die Betriebsarten
• Liveansicht-Modus,
• Vergleichs-Modus,
• Wiedergabe-Modus,
• Lösch-Modus
umfasst, wobei im Liveansicht-Modus das von der Inspektionskamera (2) aufgenommene Video direkt am LCD-Display (7) anzeigbar ist und Fotos aufnehmbar sind, wobei im Vergleichs-Modus der LCD-Display (7) in einen ersten Bereich und einen zweiten Bereich geteilt wird, wobei im ersten Bereich ein Standbild und im zweiten Bereich entweder eine Liveübertragung der Inspektionskamera (2) oder ebenfalls ein Standbild darstellbar ist, wobei im Wiedergabe-Modus die aufgenommenen Fotos am LCD-Display (7) betrachtbar sind, wobei im Lösch-Modus alle aufgenommenen Fotos gelöscht werden können, wobei vorzugsweise auch im Wiedergabe-Modus einzelne Fotos löschbar sind.

## Claims

1. Multifunction camera pocket borescope comprising
• a base body (1),
• an inspection camera (2) attached to the end of a cable (2') connected to the base body (1),
• a distance meter (3),
• an ambient temperature gauge (4),
• and a torch (6),
**characterized in that** a laser pointer (5) is provided and **in that** a liquid crystal display (LCD) (7) is mounted on the front side of the base body (1), wherein the liquid crystal display (7) comprises
• a zoom function,
• a rotation function and
• a backlight function,
wherein the zoom function preferably has four levels, wherein the rotation function preferably has four rotations, wherein the backlight function preferably has seven levels.

2. Multifunction camera pocket borescope according to claim 1, wherein the base body (1) has a connection for an external temperature sensor (4').

3. Multifunction camera pocket borescope according to any one of claims 1 or 2, wherein the base body (1) has at least one USB port (8).

4. Multifunction camera pocket borescope according to any one of claims 1 to 3, **characterized by** an integrated memory, wherein the integrated memory can preferably store up to 100 images.

5. Multifunction camera pocket borescope according to any one of claims 1 to 4, wherein a distance of preferably 5 cm to 20 m can be measured with the distance meter (3).

6. Multifunction camera pocket borescope according to any one of claims 1 to 5, wherein the ambient temperature gauge (4) is an infrared thermometer, wherein the infrared thermometer measures a temperature of -20 °C to 350 °C, wherein the measuring optics of the thermometer (4) is given by the ratio of the measuring distance to the size of the measuring spot with preferably D:S = 8:1.

7. Multifunction camera pocket borescope according to any one of claims 1 to 6, wherein the liquid crystal display (7) is a thin film transistor display, the display (7) preferably having a size of 4.3 inches and a resolution of 480 × 272 pixels.

8. Multifunction camera pocket borescope according to any one of claims 1 to 7, **characterized by** an insertion groove (9) between the front and back of the base body (1), wherein the insertion groove (9) opens by pulling the front and back of the base body (1) apart by releasing the latch (9').

9. Multifunction camera pocket borescope according to any one of claims 1 to 8, wherein the inspection camera (2) comprises LEDs, wherein the brightness of the LEDs is preferably adjustable in seven steps, wherein preferably at the first step all LEDs are off.

10. Multifunction camera pocket borescope according to any one of claims 1 to 9, wherein the inspection camera (2) and the cable (2') are stowable in the insertion groove (9).

11. Multifunction camera pocket borescope according to any one of claims 1 to 10, wherein the base body (1) of the borescope comprises the function switches
• playback / display brightness (10)
• delete / Alignment (11)
• LED work light / laser pointer (12)
• zoom / up (14)
• LED brightness / down (15)
• on / Off switch (16)
and the selector switches
• distance measurement (13)
• temperature measurement (17)
• camera shot (18).

12. Multifunction camera pocket borescope according to any one of claims 1 to 11, **characterized by** a battery compartment (19) with at least one battery for power supply, wherein preferably four AA batteries can be inserted in the battery compartment (19).

13. Use of a multi-function camera pocket borescope according to any one of claims 1 to 12, wherein the borescope is first switched on via a function switch on / off switch (16), wherein the borescope is thereafter usable in one of the modes of operation
• camera recording mode,
• temperature measurement mode,
• distance measurement mode,
• mode USB transmission.

14. Use of a multi-function camera pocket borescope according to claim 13 in the camera recording mode, which comprises the operating modes
• live view mode,
• comparison mode,
• playback mode,
• delete mode,
wherein in live view mode the video recorded by the inspection camera (2) is directly displayable on the LCD display (7) and photos are recordable, wherein in comparison mode the LCD display (7) is divided into a first area and a second area, wherein in the first area a still image can be displayed and in the second area either a live transmission of the inspection camera (2) or likewise a still image can be displayed, wherein in playback mode the recorded photos can be viewed on the LCD display (7), wherein in delete mode all recorded photos can be deleted, wherein preferably individual photos can also be deleted in playback mode.

## Revendications

1. Endoscope de poche à caméra multifonction comprenant
• un corps principal (1),
• une caméra d'inspection (2) qui est montée à l'extrémité d'un câble (2') raccordé au corps principal (1),
• un dispositif de mesure de distance (3),
• un dispositif de mesure de température ambiante (4) et
• une lampe de poche (6),
**caractérisé en ce qu'**un pointeur laser (5) est prévu et qu'un écran à cristaux liquides (LCD) (7) est monté sur la face avant du corps principal (1), lequel écran à cristaux liquides (7) comprend
• une fonction de zoom,
• une fonction de rotation et
• une fonction de rétro-éclairage,
laquelle fonction de zoom comporte de préférence quatre niveaux, laquelle fonction de rotation comporte de préférence quatre modes de rotation ou de réflexion, et laquelle fonction de rétro-éclairage comporte de préférence sept niveaux.

2. Endoscope de poche à caméra multifonction selon la revendication 1 dont le corps principal (1) dispose d'une prise pour un thermomètre externe (4').

3. Endoscope de poche à caméra multifonction selon une des revendications 1 ou 2 dont le corps principal (1) dispose d'au moins une prise USB (8).

4. Endoscope de poche à caméra multifonction selon une des revendications 1 à 3, **caractérisé par** une mémoire intégrée, laquelle mémoire intégrée peut enregistrer de préférence jusqu'à 100 images.

5. Endoscope de poche à caméra multifonction selon une des revendications 1 à 4 dont le dispositif de mesure de distance (3) permet de mesurer une distance de préférence de 5 cm à 20 m.

6. Endoscope de poche à caméra multifonction selon une des revendications 1 à 5 dont le dispositif de mesure de température ambiante (4) est un thermomètre à infrarouges, lequel thermomètre à infrarouges mesure une température de -20 °C à 350 °C, l'optique de mesure du thermomètre (4) étant donnée par le rapport de la distance de mesure à la taille de la cible de mesure avec de préférence D:S = 8:1.

7. Endoscope de poche à caméra multifonction selon une des revendications 1 à 6 dont l'écran à cristaux liquides (7) est un écran à transistors à couches minces, lequel écran présente de préférence une taille de 4,3 pouces et une définition de 480 × 272 pixels.

8. Endoscope de poche à caméra multifonction selon une des revendications 1 à 7, **caractérisé par** une rainure d'insertion (9) entre les faces avant et arrière du corps principal (1), laquelle rainure d'insertion (9) s'ouvre en séparant les faces avant et arrière du corps principal (1) par la libération du dispositif de verrouillage (9').

9. Endoscope de poche à caméra multifonction selon une des revendications 1 à 8 dont la caméra d'inspection (2) comprend des LED, la luminosité des LED étant de préférence réglable en sept niveaux, toutes les LED étant de préférence éteintes dans le premier niveau.

10. Endoscope de poche à caméra multifonction selon une des revendications 1 à 9 dont la caméra d'inspection (2) et le câble (2') peuvent être rangés dans la rainure d'insertion (9).

11. Endoscope de poche à caméra multifonction selon une des revendications 1 à 10, le corps principal (1) duquel endoscope comporte les commutateurs de fonction suivants :
• lecture/luminosité de l'écran (10)
• effacement/orientation (11)
• lampe de travail à LED/pointeur laser (12)
• zoom/haut (14)
• luminosité des LED/bas (15)
• marche/arrêt
et les sélecteurs suivants :
• mesure de distance (13)
• mesure de température (17)
• enregistrement de la caméra (18).

12. Endoscope de poche à caméra multifonction selon une des revendications 1 à 11, **caractérisé par** un compartiment à piles (19) avec au moins une pile pour l'alimentation électrique, 4 piles pouvant de préférence être insérées dans le compartiment à piles (19).

13. Utilisation d'un endoscope de poche à caméra multifonction selon une des revendications 1 à 12, lequel endoscope est d'abord allumé avec un commutateur de fonction marche/arrêt (16) et lequel endoscope peut ensuite être mis dans un des modes de fonctionnements suivants :
• mode d'enregistrement de la caméra,
• mode de mesure de température,
• mode de mesure de distance et
• mode de transmission USB.

14. Utilisation d'un endoscope de poche à caméra multifonction selon la revendication 13 en mode d'enregistrement de la caméra, lequel comprend les modes de fonctionnement
• mode d'affichage en direct,
• mode de comparaison,
• mode de lecture et
• mode d'effacement,
dans laquelle, en mode d'affichage en direct, la vidéo enregistrée par la caméra d'inspection (2) peut être affichée directement sur l'écran LCD (7) et des photos peuvent être enregistrées, dans laquelle, en mode de comparaison, l'écran LCD (7) peut être divisé en une première zone et une deuxième zone, la première zone permettant d'afficher une image fixe et la deuxième zone soit une transmission en direct de la caméra d'inspection (2), soit également une image fixe, dans laquelle, en mode de lecture, les photos enregistrées peuvent être observées sur l'écran LCD (7), et dans laquelle, en mode d'effacement, toutes les photos enregistrées peuvent être effacées, certaines photos pouvant de préférence aussi être effacées dans le mode de lecture.
